# FASCICULE DE BREVET EUROPEEN

(11) **EP 1 032 374 B1**
(45) Date de publication et mention de la délivrance du brevet: **06.08.2003**
(21) Numéro de dépôt: 98954683.3
(22) Date de dépôt: 19.11.1998
(51) Int. Cl.: A61K 9/50, A61K 9/20

(54) **SPHEROIDES, PROCEDE DE PREPARATION ET COMPOSITIONS PHARMACEUTIQUES**
SPHEROIDE, VERFAHREN ZU IHRER HERSTELLUNG UND DIESE ENTHALTENDE ARZNEIFORMEN
SPHEROIDS, PREPARATION METHOD AND PHARMACEUTICAL COMPOSITIONS

(30) Priorité: 21.11.1997 FR 9714631
(43) Date de publication de la demande: 06.09.2000
(73) Titulaire: Laboratoires des Produits Ethiques Ethypharm, 78550 Houdan (FR)
(72) Inventeur: DEBREGEAS, Patrice, F-75007 Paris (FR); LEDUC, Gérard, F-45330 Malesherbes (FR); OURY, Pascal, F-75005 Paris (FR); SUPLIE, Pascal, F-27400 Montaure (FR)
(74) Mandataire: Ahner, Francis
(86) Numéro de dépôt international: IB9801911
(87) Numéro de publication internationale: WO99026608

(56) Documents cités:
- EP-A- 0 361 874
- WO-A-96/01621
- WO-A-97/25064
- US-A- 4 684 516

## Description

La présente invention concerne une nouvelle forme galénique se présentant sous la forme de sphéroïdes, contenant un ou plusieurs principes actifs à l'exception de la tiagabine.

La présente invention s'étend en outre au procédé de préparation de tels sphéroïdes et aux préparations pharmaceutiques multiparticulaires contenant ces sphéroïdes. Ces préparations pharmaceutiques sont destinées à la délivrance des sphéroïdes qu'elles contiennent, et sont caractérisées par l'absence d'une altération du profil de libération du ou des principes actifs contenus dans les sphéroïdes.

On entend par sphéroïdes des unités sphériques dont la taille peut varier de 0,25 mm à 3 mm, de préférence de 0,5 mm à 1 mm.

Le brevet US 4 684 516 décrit des comprimés pour administration orale qui se désintègrent rapidement en milieu aqueux, qui comprennent des granules enrobés capables de libérer un principe actif à une vitesse contrôlée pendant plusieurs heures dans la voie intestinale, et qui comprennent 2 à 15% en poids d'agents liants et lubrifiants.

Le brevet US 4 684 516 décrit en particulier des granules obtenus par application d'une couche de principe actif sur des non-pareils, ces granules étant ensuite enrobés d'un premier film contenant un mélange d'acide stéarique, de cire de carnauba et de talc, et d'un deuxième film constitué d'agents désintégrants - comme l'amidon, la cellulose ou l'acide alginique - qui servent également à assurer la cohésion du comprimé.

La demande de brevet EP 468 436 décrit des comprimés à libération prolongée obtenus par compression d'un principe actif et d'un mélange de deux poudres, l'une étant hydrophobe et l'autre hydrosoluble. La poudre hydrophobe est obtenue par fusion et pulvérisation d'un mélange d'acide stéarique, de glycérine et d'huile de ricin hydrogénée. La poudre hydrosoluble est une cellulose mélangée à du lactose.

La demande de brevet EP 548 356 décrit des comprimés multiparticulaires à délitement rapide qui comprennent une substance active sous forme de monocristaux ou de microgranules.

Ces comprimés sont obtenus par granulation préalable d'un mélange d'excipients constitué d'un ou plusieurs agents de délitement, du type carboxyméthylcellulose ou polyvinylpyrrolidone, d'un ou plusieurs agents gonflants, du type amidon, et d'un sucre de compression directe comme le dextrose. Les microgranules ou les monocristaux sont intégrés à sec au mélange d'excipients avant d'être comprimés.

Il a été montré que les excipients utilisés habituellement pour enrober les granules non comprimés, comme les dérivés de cellulose, ne peuvent pas absorber normalement les contraintes mécaniques que subissent les granules lors de la compression (International Journal of Pharmaceutics, n°143, 13-23, 1996).

La compression de granules enrobés est une opération délicate car elle modifie la structure du film d'enrobage par l'apparition de fissures ou par rupture, ce qui conduit à la perte partielle ou totale des propriétés du film.

La fissuration des granules modifie irréversiblement le profil de libération du ou des principes actifs qu'ils contiennent.

De façon à conserver les caractéristiques du film d'enrobage des granules après compression, les granules de l'art antérieur sont dilués avec des substances auxiliaires, dont le rôle est d'absorber les contraintes physiques liées à la compression (agents liants) et de permettre la désagrégation du comprimé (agents désintégrants) en milieu liquide, i.e. en solution aqueuse ou dans le fluide digestif.

Les formulations de comprimés de l'art antérieur utilisent des substances auxiliaires ajoutées aux granules lors de la compression pour éviter la fissuration de ces granules en surface.

L'objet de la présente invention concerne des sphéroïdes contenant un ou plusieurs principes actifs à l'exception de la tiagabine, compressibles directement, sans l'ajout d'une partie substantielle d'une substance auxiliaire, i.e. moins de 5 % en poids, de préférence moins de 1 % en poids.

La présente invention a pour objet des sphéroïdes contentant un ou plusieurs principes actifs, à l'exception de la tiagabine, qui comprennent :
- un noyau et une couche enrobant ledit noyau, tels que le noyau et/ou la couche enrobante contient au moins un excipient thermoplastique, dont la consistance est pâteuse à semi-solide à une température de 20°C, et dont la température de fusion est comprise entre 25°C et 100°C, enrobé d'
- un film souple et déformable c'est-à-dire présentant un pourcentage d'élongation supérieur à 50 %, à base d'un matériau polymérique en particulier un film dont la température de transition vitreuse est inférieure à 30°C, qui assure, soit la protection, soit le masquage du goût, soit la libération contrôlée et modifiée du ou des principes actifs.

Le noyau peut être composé en particulier d'un mélange de saccharose et d'amidon ou de cellulose microcristalline.

Dans le cadre de la présente invention, on entend par excipients thermoplastiques des composés ayant un point de fusion compris entre 25 et 100°C et caractérisés par une consistance pâteuse à semi-solide à une température de 20°C.

Le rôle des excipients thermoplastiques est, en particulier, lors d'une éventuelle étape de compression, de permettre aux sphéroïdes de se déformer plastiquement, et ainsi d'absorber une partie des contraintes qu'ils subissent, si bien que leur surface n'est pas fissurée ou déchirée.

Les excipients peuvent être avantageusement choisis parmi les huiles partiellement hydrogénées, la cire d'abeille, la cire de carnauba, les cires de paraffine, les cires de silicone, les alcools gras et les acides gras en C12-C18, les glycérides semi-synthétiques solides, les monoester-diesters ou triesters du glycérol, les polyoxyéthylèneglycols, les glycérides polyoxyéthylénés glycosylés, et leurs mélanges.

La couche contenant au moins un excipient thermoplastique est enrobée d'un film souple et déformable, comprenant un matériau polymérique, dont la température de transition vitreuse est inférieure à 30°C, de préférence inférieure à 20°C.

Le film polymérique comprend un matériau. polymérique qui est, soit un polymère, soit un mélange d'au moins un polymère et d'un plastifiant.

Le film polymérique peut servir selon les cas à protéger le principe actif vis à vis de l'environnement (dégradation par la lumière, par l'humidité ambiante), à masquer le goût du principe actif, ou à modifier sa libération (libération prolongée, retardée ou programmée).

Le polymère est de préférence un polymère ou un copolymère acrylique, vinylique ou cellulosique.

On entend par plastifiant un produit permettant de diminuer la température de transition vitreuse du polymère.

Le plastifiant est de préférence choisi parmi le triéthyl citrate, l'acétyl triéthyl citrate, le tributyl citrate, l'acétyl tributyl citrate, la triacétine, le diéthyl phtalate, les polyéthylène glycols, les polysorbates, les glycérides *mono-* et diacétylés, et leurs mélanges.

Les propriétés mécaniques du film polymérique, en particulier le pourcentage d'élongation et la résistance à la rupture, peuvent servir de critères de sélection du polymère et/ou du plastifiant. Ces caractéristiques mécaniques peuvent en outre être déterminées par la méthode décrite dans les normes DIN53 455 et ISO/RI 184.

A titre d'exemple le polymère Eudragit NE30D® commercialisé par la société RÖHM, qui est un copolymère neutre d'esters d'acides acrylique et méthacrylique sous forme d'une dispersion aqueuse à 30%, présente un pourcentage d'élongation de 600% et une résistance à la rupture égale à 8 N/mm² ce qui le rend particulièrement souple et déformable.

Selon une première variante de l'invention, le ou les principes actifs sont dispersés dans la masse du noyau.

Selon une deuxième variante, le ou les principes actifs sont dispersés dans la couche contenant au moins un excipient thermoplastique.

Selon une troisième variante, le ou les principes actifs sont appliqués à la surface du noyau, puis recouverts d'une couche contenant au moins un excipient thermoplastique.

Le principe actif peut être protégé, dans l'une des quatre variantes précédentes, en lui associant un agent antioxydant et/ou en l'enrobant d'un film de protection.

Enfin, selon une quatrième et dernière variante, le ou les principes actifs sont dispersés dans la masse du noyau et dans la couche contenant au moins un excipient thermoplastique.

Les sphéroïdes selon l'invention peuvent avantageusement être enrobés d'une couche externe hydrodispersible.

La couche externe assure la cohésion desdits sphéroïdes lors d'une éventuelle étape de compression, et assure le délitement en milieu aqueux du comprimé obtenu.

La couche externe hydrodispersible est de préférence constituée d'un polymère acrylique, vinylique ou cellulosique.

La présente invention a également pour objet le procédé de préparation des sphéroïdes décrits précédemment.

La fabrication des noyaux peut être réalisée par montage en turbine à partir de cristaux calibrés de saccharose, ou par extrusion-sphéronisation.

Lorsque les noyaux sont préparés par extrusion-sphéronisation, le principe actif peut être mélangé à la masse extrudée-sphéronisée.

Selon le procédé de préparation de l'invention la couche contenant au moins un excipient thermoplastique, le film contenant un matériau polymérique, et éventuellement la couche de protection externe sont successivement déposés sur les noyaux par pulvérisation, dans une turbine de granulation, dans une turbine perforée, en lit d'air fluidisé ou par tout autre moyen approprié.

Le procédé selon l'invention comprend deux ou trois étapes selon que l'on souhaite préparer des sphéroïdes comportant une couche externe hydrodispersible ou non.

Les sphéroïdes comportant une couche externe hydrodispersible sont particulièrement adaptés à la préparation de comprimés.

La première étape, appelée étape de montage, consiste à déposer le ou les excipients thermoplastiques sur les noyaux. La préparation de montage peut se présenter, selon les cas, sous forme de dispersions solides en milieux aqueux ou organiques, sous forme de solutions, sous forme d'émulsions, ou à l'état fondu.

Lorsque le principe actif est dispersé dans la masse de la couche contenant au moins un excipient thermoplastique, le principe actif est incorporé à la préparation de montage.

Selon une autre variante du procédé selon l'invention, le principe actif peut être fixé par poudrage sur les noyaux neutres préalablement mouillés avec la préparation de montage.

La deuxième étape du procédé selon l'invention consiste à déposer le film polymérique permettant d'assurer la protection du principe actif, le masquage de goût ou la libération modifiée du principe actif. La préparation d'enrobage polymérique peut se présenter sous forme de solutions ou de dispersions en milieu aqueux ou organique.

La troisième étape facultative du procédé selon l'invention consiste à déposer la couche externe de protection en pulvérisant la préparation d'enrobage qui peut se présenter sous forme de solution ou de dispersion en milieu aqueux ou organique.

En fonction des besoins, on peut avantageusement ajouter aux préparations de montage et d'enrobage un agent anti-adhérant tel que le talc, un plastifiant tel que le polyéthylène glycol, un agent antioxydant tel que le dl-alpha-tocophérol.

A la couche externe de protection on peut avantageusement ajouter un agent désintégrant de façon à accélérer la libération des sphéroïdes en milieu aqueux, lorsque ceux-ci ont été comprimés.

L'agent désintégrant est par exemple la carboxyméthylcellulose réticulée, la polyvinylpyrrolidone réticulée ou le carboxyméthyl amidon sodique.

La présente invention a également pour objet les préparations pharmaceutiques multiparticulaires contenant les sphéroïdes décrits précédemment susceptibles d'être obtenus par le procédé de l'invention.

Les préparations pharmaceutiques multiparticulaires selon l'invention sont de préférence sans la forme de gélules remplies desdits sphéroïdes, ou de comprimés desdits sphéroïdes.

Lesdits comprimés sont avantageusement préparés sans l'addition substantielle de substances auxiliaires. On peut ajouter aux sphéroïdes avant la compression, jusqu'à 5 % en poids d'un agent lubrifiant comme le talc.

Lesdits comprimés comprennent avantageusement des de sphéroïdes protégés par une couche hydrodispersible constituée par un polymère acrylique, vinylique, cellulosique ou un excipient thermoplastique hydrodispersible, ou tout autre excipient soluble en milieu aqueux.

Cette couche a pour rôle d'assurer la cohésion des sphéroïdes entre eux, d'assurer ainsi la dureté du comprimé, et de permettre au comprimé de se désagréger lorsqu'il est plongé en solution.

Les comprimés selon l'invention sont dispersibles en solution et restituent des sphéroïdes indépendants, de sorte que le profil de libération du comprimé et des sphéroïdes qui le constituent sont pratiquement équivalents.

En effet, les comprimés selon l'invention permettent la délivrance de sphéroïdes sans que le profil de libération du ou des principes actifs qu'ils contiennent ne soit altéré sous l'effet de la compression.

Les comprimés selon l'invention peuvent être composés uniquement des sphéroïdes selon l'invention ou d'un mélange de sphéroïdes et de sphéroïdes placebos, c'est-à-dire des sphéroïdes conforment à la présente invention mais dépourvus de principe actif.

Les contraintes exercées sur les sphéroïdes, lors de l'étape de compression, peuvent varier de 5 kN à 50 kN mais de préférence entre 5 kN et 15 kN.

La dureté des comprimés est de préférence comprise entre 10 N et 100 N, plus préférentiellement entre 10 N et 50 N.

Le temps de désagrégation des comprimés en milieu aqueux à 37°C est inférieur à 60 min.

Les comprimés selon l'invention ont de préférence une masse comprise entre 0,1 g et 1 g.

Leur forme peut être ronde, ovale, oblongue, présenter une surface plate ou concave, et présenter des gravures ou des barres de sécabilité.

Les comprimés selon l'invention peuvent faire l'objet d'un enrobage final de protection ou de coloration.

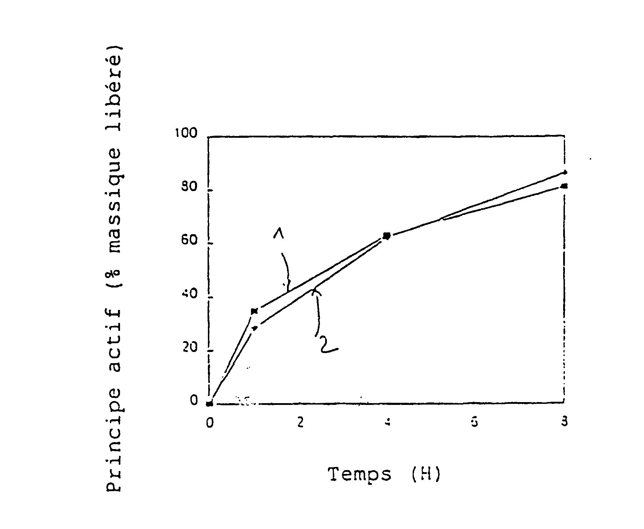

La présente invention sera plus particulièrement décrite à l'aide des exemples suivants et de la figure unique qui ne doivent pas être considérés comme limitatifs de l'invention.

La figure unique représente le pourcentage massique de dissolution in vitro d'un principe actif particulier, l'isosorbide mononitrate, pour les sphéroïdes selon l'invention non comprimés (courbe 1) et pour un comprimé de sphéroïdes selon l'invention (courbe 2).

### EXEMPLE 1

### Etape de montage

- Préparer une solution aqueuse de montage contenant le principe actif et de l'hydroxypropyl-méthyl-cellulose (Pharmacoat 603® commercialisé par la société SHIN ETSU),
- Dans une turbine perforée, pulvériser la solution décrite précédemment à la surface de Neutres 26® (commercialisés par NP Pharm) de façon à obtenir une enveloppe composée de Pharmacoat 603® et de principe actif,
- Transférer la charge ainsi obtenue dans une turbine conventionnelle; chauffer la charge jusqu'à une température d'environ 50°C,
- Appliquer par poudrage sur la charge chaude en rotation du Précirol® atomisé (commercialisé par la société Gattefossé). Le Précirol® est un composé thermoplastique qui, par ramollissement et étalement au contact de la charge maintenue à 50°C, va former un film uniforme autour des noyaux.
- Pour réduire le collage entre les noyaux, du talc peut être ajouté au Précirol®.

### Etape d'enrobage

La charge obtenue précédemment est divisée en deux. Une partie seulement est enrobée:
- Préparer une solution aqueuse composée d'un polymère acrylique, I'Eudragit RS30D® (commercialisé par la société RÖHM) et d'un plastifiant le Myvacet 9.45® (commercialisé par la société Eastman Kodak),
- Dans une turbine perforée, pulvériser la solution d'enrobage sur les noyaux montés,
- Sécher la charge ainsi obtenue.

### Etape de mélange

- mélanger dans les proportions massiques 27/73 la partie enrobée et la partie montée; le détail des compositions des deux lots et du mélange apparaît ci-dessous:

| Composition | Noyaux montés(g) | Noyaux enrobés(g) | Mélange(g) | Mélange (%) |
|---|---|---|---|---|
| Neutre 26® | 25,4 | 59,2 | 84,6 | 56,1 |
| Isosorbide Mononitrate (Principe actif) | 5,9 | 13,8 | 19,7 | 13,1 |
| Pharmacoat 603® | 1,0 | 2,2 | 3,2 | 2,1 |
| Précirol® | 6,5 | 15,1 | 21,6 | 14,3 |
| Talc | 1,3 | 3,0 | 4,3 | 2,9 |
| Eudragit RS30D® | - | 14,4 | 14,4 | 9,6 |
| Myvacet 9.45® | - | 2,9 | 2,9 | 1,9 |
| Total | 40,1 | 110,6 | 150,7 | 100,0 |

### Etape de compression

Le mélange de sphéroïdes est comprimé sur une presse alternative Frogerais OA.

### Résultats de dissolution in vitro:

Un test de dissolution in vitro est réalisé sur les comprimés selon la méthode décrite dans la pharmacopéee (USP XXIII, <711> appareil 2).

### Conditions :

- appareil à palette tournante,
- milieu : pH 6,0, 500 ml, 37°C.

Le principe actif est dosé par spectrophotométrie ultra-violet.

Le tableau suivant donne le pourcentage de principe actif libéré par les sphéroïdes avant et après compression en fonction du temps:

| Temps (h) | Sphéroïdes Pourcentage massique d' Isosorbide Mononitrate libéré (%) | Comprimés dispersibles Pourcentage massique d' Isosorbide Mononitrate libéré (%) |
|---|---|---|
| 0 | 0 | 0 |
| 1 | 28,2 | 34,8 |
| 4 | 62,2 | 63,2 |
| 8 | 86,4 | 80,9 |

Les résultats présentés sur la figure unique montrent qu'il n'y a pas de différence significative entre les profils de dissolution du principe actif avant et après compression.

### EXEMPLE 2

### Etape de montage

### Préparation de la suspension de montage

Peser les excipients dans les proportions indiquées ci-dessous.

| Matières | Quantité(g) | Pourcentage |
|---|---|---|
| CODEÏNE | 100,0 | 52,1 % de l'extrait sec |
| Cire Novata AB® | 38,0 | 19,8 % de l'extrait sec |
| PEG 6000 | 13,0 | 6,8 % de l'extrait sec |
| Polysorbate 80 | 6,0 | 3,1 % de l'extrait sec |
| dl-α-tocophérol | 10,0 | 5,2 % de l'extrait sec |
| Talc | 25,0 | 13,0 % de l'extrait sec |
| Eau purifiée | 233,0 | 100 % du solvant |
| Teneur en extrait sec : 45,2 % | | |

- Chauffer l'eau purifiée à 37°C,
- Ajouter le PEG 6000 (Empakol® - commercialisé par ICI) et le faire fondre jusqu'à obtenir une solution homogène,
- Chauffer la cire Novata AB® (commercialisée par Henkel) à 37°C,
- Ajouter à la cire le polysorbate 80 (commercialisé par ICI) et le dl-α-tocophérol (commercialisé par Roche),
- Mélanger à 37°C, à l'aide d'un agitateur de type Heidolph la solution aqueuse et la solution huileuse afin d'obtenir une émulsion de type huile dans eau,
- Refroidir de façon à ramener la température aux environs de 25°C,
- Ajouter le principe actif,
- Broyer la suspension à l'aide d'une turbine de type Ultra-Turrax®,
- Ajouter en final le talc et maintenir la suspension sous agitation pendant l'opération de montage.

### Montage sur des noyaux neutres

- Placer 550 g de neutres taille 30 (commercialisé par NP-PHARM), dans une turbine à dragéification, une turbine perforée ou un lit d'air fluidisé,
- Effectuer le montage par pulvérisation en continue de la suspension décrite ci-dessus,
- Maintenir la température entre 20 et 23°C pendant toute l'opération,
- Tamiser la masse de microgranules obtenue.

### Formule finale :

| Matières | Pourcentage |
|---|---|
| Neutres 30® | 74,1 % |
| CODEÏNE | 13,5 % |
| Cire Novata AB® | 5,1 % |
| PEG 6000 | 1,8 % |
| Polysorbate 80 | 0,8 % |
| dl-α-tocophérol | 1,3 % |
| Talc | 3,4 % |
| Teneur théorique en CODEINE | 135 mg/g |

### Etape d'enrobage

### 1 / Préparation de la suspension d'enrobage à libération prolongée

- Peser les excipients d'enrobage suivants dans les proportions indiquées :

| Matières | Quantité(g) | Pourcentage |
|---|---|---|
| Eudragit NE 30D® | 40,0 | 80,0 % d'extrait sec |
| PEG 6000 | 10,0 | 20,0 % d'extrait sec |
| Eau purifiée | 158,0 | 100 % du solvant |
| Teneur en extrait sec : 24,0 % | | |

- Placer l'eau purifiée dans un récipient sous agitation,
- Solubiliser le PEG 6000 jusqu'à obtention d'une solution homogène,
- Ajouter lentement l'Eudragit NE 30D® (commercialisé par la société RÖHM), agiter jusqu'à obtenir une suspension homogène,
- Maintenir l'agitation pendant toute la phase d'enrobage.

### Enrobage des sphéroïdes montés

- Placer une fraction des microgranules obtenus selon l'exemple 1 dans une turbine perforée ou un lit d'air fluidisé,
- Effectuer l'enrobage de sphéroïdes montés par pulvérisation continue de la suspension décrite ci-dessus en maintenant le lit de sphéroïdes à une température inférieure à 25°C,
- Sécher puis tamiser la masse ainsi obtenue.

### 2 / Préparation de la suspension d'enrobage de protection

- Peser les excipients d'enrobage suivants dans les proportions indiquées :

| Matières | Quantité (g) | Pourcentage |
|---|---|---|
| Opadry OYB® | 95,0 | 95,0 % d'extrait sec |
| PEG 6000 | 5,0 | 5,0 % d'extrait sec |
| Eau purifiée | 1000 | 100 % du solvant |
| Teneur en extrait sec : 10,0 % | | |

- Placer l'eau purifiée dans un récipient sous agitation,
- Solubiliser le PEG 6000 jusqu'à obtention d'une solution homogène,
- Ajouter lentement l'Opadry OYB® (commercialisé par la société COLORCON), agiter jusqu'à obtenir une solution homogène,
- Maintenir l'agitation pendant toute la phase d'enrobage.

### Enrobage des sphéroïdes à libération prolongée

- Placer une fraction des sphéroïdes obtenus précédemment dans une turbine perforée ou un lit d'air fluidisé,
- Effectuer l'enrobage de sphéroïdes à libération prolongée par pulvérisation continue de la solution décrite ci-dessus en maintenant le lit de sphéroïdes à une température inférieure à 25°C,
- Sécher à 30°C/35°C en fin d'enrobage puis tamiser la masse ainsi obtenue.
Formule finale correspondant à 35 % p/p d'enrobage à libération prolongée et à 5 % p/p d'enrobage de protection :

| Matières | Pourcentage |
|---|---|
| Neutres 30® | 44,9 % |
| CODEÏNE | 8,2 % |
| Cire Novata AB® | 3,1 % |
| PEG 6000 | 8,3 % |
| Polysorbate 80® | 0,5 % |
| dl-α-tocophérol | 0,8 % |
| Talc | 2,1 % |
| Opadry OYB® | 4,1 % |
| Eudragit NE 30D® | 28,0 % |
| Teneur théorique en CODEINE | 82 mg /g |

Les sphéroïdes sont ensuite comprimés sur une machine instrumentée rotative de type Fette P1200. Les forces de compression appliquées se situent entre 10 kN et 30 kN.

Caractéristiques des comprimés mesurées sur 10 unités :

| Dosage | Poids moyen | Taille 1xLxh (mm) | Dureté | Friabilité | Désagrégation |
|---|---|---|---|---|---|
| 29,93 mg/g | 365 mg | 12,5x7,5x4,8 | 20 N | < 0,1 % | 45 min |

### Résultats de dissolution in vitro :

### Conditions :

- appareil de dissolution à palette conforme à la norme USP XXIII, <711> appareil 2,
- milieu : 900 ml d'eau purifiée à 37°C agité à 100 tours/minutes.

Le principe actif est dosé par HPLC à la longueur d'onde 285 nm.

Le tableau suivant donne le pourcentage de principe actif libéré par les sphéroïdes avant et après compression en fonction du temps.

| Temps (h) | Sphéroïdes Pourcentage de CODEÏNE libérée | Comprimés dispersibles Pourcentage de CODEÏNE libérée |
|---|---|---|
| 1 | 20,5 | 24,5 |
| 2 | 45,9 | 52,3 |
| 3 | 63,9 | 73,4 |
| 4 | 75,4 | 85,0 |
| 6 | 89,4 | 94,4 |
| 8 | 90,5 | 97,1 |

### EXEMPLE 3

### Etape de montage

### Préparation de la suspension de montage

Peser les excipients dans les proportions indiquées ci-dessous

| Matières | Quantité (g) | Pourcentage (%) |
|---|---|---|
| Sulfate de morphine | 183,54 | 19,77 |
| Diéthylphtalate | 27,57 | 2,97 |
| Talc | 13,76 | 1,48 |
| Eau | 703,68 | 75,98 |
| Teneur en extrait sec :24,22 % | | |

### Mode opératoire

Dans un récipient Inox, introduire successivement l'eau purifiée puis sous agitation le principe actif par petites quantités. Ajouter progressivement toujours sous agitation le diéthylphtalate puis le talc.
Mélanger la suspension à l'aide d'une turbine ultra-Turrax®.

### Montage sur noyaux Neutres

Placer 153 g de Neutres 26® dans une turbine à dragéification ou turbine perforée au lit d'air fluidisé.
Effectuer le montage du principe actif par pulvérisation continue de la suspension décrite ci-dessus.

### Enrobage protecteur

Peser dans un récipient Inox les excipients suivants :
Precirol® : 36,85 g
Talc : 14,74 g.

Procéder au mélange de poudres (utilisation d'un mélangeur cubique ou planétaire).

### Application du mélange sur les granules

- Introduire dans la cuve de l'appareil retenu (turbine conventionnelle, lit d'air fluidisé, turbine perforée) les granules actifs.
- Réchauffer ces microgranules jusqu'à obtention d'une température d'environ 50°C.
- Appliquer par poudrage le mélange ci-dessus décrit et dans le cas d'utilisation de la technologie du lit d'air fluidisé, par pulvérisation du mélange fondu.

### Enrobage primaire

- peser dans un récipient Inox les excipients ci-dessus

| Matières | Quantité (g) | Pourcentage |
|---|---|---|
| Endragit NE 30D® | 180,74 g | 65,1 |
| Talc | 5,6 g | 2,0 |
| Eau | 91,3 g | 32,9 |
| Teneur en extrait sec : 21,5 %. | | |

- Mettre sous agitation et homogénéiser à l'aide d'une turbine Ultra-Turrax® la suspension décrite ci-dessus.
- Effectuer l'enrobage des granules décrits précédemment par pulvérisation continue de la suspension préparée ci-dessus en maintenant, quelle que soit la technologie retenue, une température du lit de granules inférieure à 23°C.

### Enrobage secondaire

- Peser dans un récipient Inox les excipients cidessous.

| Matières | Quantité (g) | Pourcentage |
|---|---|---|
| Pharmacoat 603® | 14,4 | 7,4 |
| PEG 400 | 1,5 | 0,77 |
| Eau | 178,1 | 91,8 |

- Incorporer successivement dans l'eau, le PEG puis le Pharmacoat® sous agitation et maintenir celle-ci jusqu'à complète dissolution.
- Effectuer l'enrobage des granules décrits précédemment par pulvérisation continue de la solution préparée ci-dessus.

### Formule finale

| Matières | Pourcentage massique |
|---|---|
| Neutres 26® | 30,28 |
| Sulfate de morphine | 36,33 |
| Diéthylphtalate | 5,46 |
| Talc | 6,74 |
| Précirol® | 7,29 |
| Eudragit NE 30D® | 10,73 |
| Pharmacoat 603® | 2,85 |
| PEG 400 | 0,29 |
| Teneur théorique en sulfate de morphine : 235,67 mg/g. | |

Les sphéroïdes sont ensuite comprimés sur une machine instrumentée rotative de type Fette P1200.

Les forces de compression appliquées se situent entre 10 KN et 30 KN.

### Caractéristiques des comprimés obtenus

| Dosage | Poids moyen | Taille 1xLxh | Dureté |
|---|---|---|---|
| 99,8 mg/g | 423,5 mg | 12,5 x 7,5 x 5,2 | 19 |

### Résultats de dissolution in vitro.

### Conditions :

Appareil de dissolution à palettes conformes à la norme (USP XXIII, <711> appareil 2).
Milieu 500 ml, Eau purifiée à 37°C.
Vitesse d'agitation 100 tours/minute.

Le principe actif est dosé par U.V en continu aux longueurs d'onde : 285-320 nm.

| Temps | Sphéroïdes Pourcentage de sulfate de morphine libéré | Comprimés Pourcentage de sulfate de morphine libéré |
|---|---|---|
| 0 | 0 | 0 |
| 1 | 8 | 13 |
| 2 | 32 | 30 |
| 3 | 51,4 | 46 |
| 4 | 64,5 | 65,2 |
| 6 | 79,8 | 90,2 |
| 8 | 88 | 96 |

## Revendications

1. Sphéroïdes contenant un ou plusieurs principes actifs, à l'exception, de la tiagabine, qui comprennent :
- un noyau et une couche enrobant ledit noyau, tels que le noyau et/ou la couche enrobante contient au moins un excipient thermoplastique, dont la consistance est pâteuse à semi-solide à une température de 20°C, et dont la température de fusion est comprise entre 25°C et 100°C , enrobé d'
- un film souple et déformable c'est-à-dire présentant un pourcentage d'élongation supérieur à 50 % déterminé par la méthode décrite dans la norme DIN 53455, à base d'un matériau polymérique en particulier un film dont la température de transition vitreuse est inférieure à 30°C, qui assure, soit la protection, soit le masquage du goût, soit la libération contrôlée et modifiée du ou des principes actifs, lesquels sphéroïdes peuvent être compressés directement en ajoutant moins de 5 % en poids d'une substance auxiliaire.

2. Sphéroïdes selon la revendication 1, **caractérisés en ce que** l'excipient thermoplastique est choisi parmi les huiles partiellement hydrogénées, la cire d'abeille, la cire de carnauba, les cires de paraffine, les cires de silicone, les alcools gras et les acides gras en C12-C18, les glycérides semi-synthétiques solides, les monoester-diesters ou triesters du glycérol, les polyoxyéthylèneglycols, les glycérides polyoxyéthylénés glycosylés, et leurs mélanges.

3. Sphéroïdes selon la revendication 1 ou 2, **caractérisés en ce que** la température de transition vitreuse du film souple et déformable est inférieure à 20°C.

4. Sphéroïdes selon l'une des revendications 1 à 3, **caractérisés en ce que** le matériau polymérique est un polymère ou un mélange d'au moins un polymère et d'un plastifiant.

5. Sphéroïdes selon la revendication 4, **caractérisés en ce que** le plastifiant est choisi parmi le triéthyl citrate, l'acétyl triéthyl citrate, le tributyl citrate, l'acétyl tributyl citrate, la triacétine, le diéthyl phtalate, les polyéthylèneglycols, les polysorbates, les glycérides mono- et diacétylés, et leurs mélanges.

6. Sphéroïdes selon l'une des revendications précédentes, **caractérisés en ce qu'**ils sont enrobés d'une couche externe hydrodispersible qui assure la cohésion desdits sphéroïdes lors d'une éventuelle étape de compression et qui assure le délitement en milieu aqueux du comprimé obtenu.

7. Sphéroïdes selon la revendication 6, **caractérisés en ce que** la couche externe hydrodispersible est constituée d'un polymère acrylique, vinylique ou cellulosique.

8. Sphéroïdes selon l'une des revendications 1 à 7, **caractérisés en ce que** le ou les principes actifs sont dispersés dans la masse du noyau.

9. Sphéroïdes selon l'une des revendications 1 à 7, **caractérisés en ce que** le ou les principes actifs sont dispersés dans la couche contenant au moins un excipient thermoplastique.

10. Sphéroïdes selon l'une des revendications 1 à 7, **caractérisés en ce que** le ou les principes actifs sont dispersés dans la masse du noyau et dans la couche contenant au moins un excipient thermoplastique.

11. Sphéroïdes selon l'une des revendications 1 à 7, **caractérisés en ce que** le ou les principes actifs sont appliqués à la surface du noyau, puis recouverts d'une couche contenant au moins un excipient thermoplastique.

12. Procédé de préparation des sphéroïdes selon l'une des revendications 1 à 11, **caractérisé en ce que** la couche contenant au moins un excipient thermoplastique, le film contenant un matériau polymérique, et éventuellement la couche externe, sont successivement déposés sur les noyaux par pulvérisation, dans une turbine de granulation, dans une turbine perforée, en lit d'air fluidisé ou par tout autre moyen approprié.

13. Préparations pharmaceutiques multiparticulaires contenant les sphéroïdes selon l'une des revendications 1 à 11 susceptibles d'être obtenus selon le procédé de la revendication 12.

14. Préparations pharmaceutiques selon la revendication 13, **caractérisées en ce qu'**elles sont sous la forme de gélules remplies desdits sphéroïdes.

15. Préparations pharmaceutiques selon la revendication 13, **caractérisées en ce qu'**elles sont sous la forme de comprimés desdits sphéroïdes.

16. Procédé de fabrication des préparations selon la revendication 15, **caractérisé en ce que** les comprimés sont préparés sans l'ajout d'une quantité substantielle d'une substance auxiliaire, notamment moins de 5 % en poids.

17. Procédé selon la revendication 16, **caractérisé en ce qu'**on ajoute aux sphéroïdes avant la compression, jusqu'à 5% en poids d'un agent lubrifiant comme le talc.

## Patentansprüche

1. Sphäroide, die einen oder mehrere Wirkstoffe mit Ausnahme von Tiagabin enthalten, die umfassen:
- einen Kern und eine den Kern umhüllende Schicht, derart, dass der Kern und/oder die umhüllende Schicht wenigstens eine thermoplastische Trägersubstanz, deren Konsistenz bei einer Temperatur von 20°C pastenartig bis halbfest ist und deren Schmelztemperatur zwischen 25°C und 100°C liegt, enthält, umhüllt von
- einem elastischen und deformierbaren Film, d.h. welcher einen Dehnungsprozentsatz über 50%, bestimmt durch die in der Norm DIN 53455 beschriebene Methode, aufweist, auf der Grundlage eines polymeren Materials, insbesondere einem Film, dessen Glasübergangstemperatur unter 30°C liegt, welcher entweder den Schutz oder die Maskierung des Geschmacks oder die kontrollierte und modifizierte Freisetzung des oder der Wirkstoffe sicherstellt, wobei die Sphäroide direkt verdichtet werden können, indem man weniger als 5 Gew.-% einer Hilfssubstanz zusetzt.

2. Sphäroide nach Anspruch 1, **dadurch gekennzeichnet, dass** die thermoplastische Trägersubstanz unter den partiell hydrierten Ölen, Bienenwachs, Carnaubawachs, den Paraffinwachsen, den Siliconwachsen, den Fettalkoholen und den C12-C18-Fettsäuren, den halbsynthetischen festen Glyceriden, den Monoester-Diestern oder Triestern von Glycerol, den Polyoxyethylenglycolen, den glycosylierten Glyceriden mit Polyoxyethyleneinheiten und deren Mischungen ausgewählt wird.

3. Sphäroide nach Anspruch 1 oder 2, **dadurch gekennzeichnet, dass** die Glasübergangstemperatur des elastischen und deformierbaren Films unter 20°C liegt.

4. Sphäroide nach einem der Ansprüche 1 bis 3, **dadurch gekennzeichnet, dass** das polymere Material ein Polymer oder eine Mischung von wenigstens einem Polymer und einem Weichmacher ist.

5. Sphäroide nach Anspruch 4, **dadurch gekennzeichnet, dass** der Weichmacher unter Triethylcitrat, Acetyltriethylcitrat, Tributylcitrat, Acetyltributylcitrat, Triacetin, Diethylphthalat, den Polyethylenglycolen, den Polysorbaten, den mono- und diacetylierten Glyceriden und deren Mischungen ausgewählt wird.

6. Sphäroide nach einem der vorangegangenen Ansprüche, **dadurch gekennzeichnet, dass** sie von einer äußerlichen, in Wasser dispergierbaren Schicht umhüllt sind, die den Zusammenhalt der Sphäroide während eines gegebenenfalls erfolgenden Verdichtungsschritts sicherstellt und die den Zerfall der erhaltenen Tablette in wässrigem Medium sicherstellt.

7. Sphäroide nach Anspruch 6, **dadurch gekennzeichnet, dass** die äußerliche, in Wasser dispergierbare Schicht aus einem acrylischen, vinylischen oder cellulosischen Polymer gebildet wird.

8. Sphäroide nach einem der Ansprüche 1 bis 7, **dadurch gekennzeichnet, dass** der oder die Wirkstoffe in der Masse des Kerns dispergiert sind.

9. Sphäroide nach einem der Ansprüche 1 bis 7, **dadurch gekennzeichnet, dass** der oder die Wirkstoffe in der Schicht, die wenigstens eine thermoplastische Trägersubstanz enthält, dispergiert sind.

10. Sphäroide nach einem der Ansprüche 1 bis 7, **dadurch gekennzeichnet, dass** der oder die Wirkstoffe in der Masse des Kerns und in der Schicht, die wenigstens eine thermoplastische Trägersubstanz enthält, dispergiert sind.

11. Sphäroide nach einem der Ansprüche 1 bis 7, **dadurch gekennzeichnet, dass** der oder die Wirkstoffe auf die Oberfläche des Kerns aufgetragen, dann von einer Schicht, die wenigstens eine thermoplastische Trägersubstanz enthält, bedeckt werden.

12. Verfahren zur Herstellung der Sphäroide nach einem der Ansprüche 1 bis 11, **dadurch gekennzeichnet, dass** die Schicht, die wenigstens eine thermoplastische Trägersubstanz enthält, der Film, der ein polymeres Material enthält, und gegebenenfalls die äußerliche Schicht nacheinander auf den Kernen durch Zerstäubung, in einer Granulierturbine, in einer perforierten Turbine, in einer luftdurchströmten Wirbelschicht oder durch ein jegliches anderes geeignetes Mittel abgeschieden werden.

13. Eine Mehrzahl von Teilchen umfassende pharmazeutische Präparate, die die Sphäroide nach einem der Ansprüche 1 bis 11, die gemäß dem Verfahren nach Anspruch 12 erhalten werden können, enthalten.

14. Pharmazeutische Präparate nach Anspruch 13, **dadurch gekennzeichnet, dass** sie in Form von Kapseln, die mit den Sphäroiden gefüllt sind, vorliegen.

15. Pharmazeutische Präparate nach Anspruch 13, **dadurch gekennzeichnet, dass** sie in Form von Tabletten oder Presslingen aus den Sphäroiden vorliegen.

16. Verfahren zur Herstellung der Präparate nach Anspruch 15, **dadurch gekennzeichnet, dass** die Tabletten oder Presslinge ohne Zugabe einer substantiellen Menge einer Hilfssubstanz, insbesondere weniger als 5 Gew.-%, hergestellt werden.

17. Verfahren nach Anspruch 16, **dadurch gekennzeichnet, dass** man zu den Sphäroiden vor dem Verdichten bis zu 5 Gew.-% eines Gleitmittels, wie Talkum, zusetzt.

## Claims

1. Spheroids containing one or more active principles, with the exception of tiagabine, which comprise:
- a core and/or a layer coating the said core, such that the core and/or the coating layer contain at least one thermoplastic excipient which is of pasty to semi-solid consistency at a temperature of 20°C, and whose melting point is between 25°C and 100°C, coated with
- a flexible and deformable film, i.e. having a percentage of elongation of greater than 50%, determined using the method described in standard DIN 53455, based on a polymer material, in particular a film whose glass transition temperature is less than 30°C, which ensures either protection or masking of the taste, or controlled and modified release of the active principle(s), which speroids can be compressed directly, adding less than 5% by weight of an auxiliary substance.

2. Spheroids according to Claim 1, **characterized in that** the thermoplastic excipient is chosen from partially hydrogenated oils, beeswax, carnauba wax, paraffin waxes, silicone waxes, C12-C18 fatty alcohols and fatty acids, solid, semi-synthetic glycerides, glycerol monoesters, diesters or triesters, polyoxyethylene glycols and glycosylated polyoxyethylenated glycerides, and mixtures thereof.

3. Spheroids according to Claim 1 or 2, **characterized in that** the glass transition temperature of the flexible and deformable film is less than 20°C.

4. Spheroids according to one of Claims 1 to 3, **characterized in that** the polymer material is a polymer or a mixture of at least one polymer and a plasticizer.

5. Spheroids according to Claim 4, **characterized in that** the plasticizer is chosen from triethyl citrate, acetyl triethyl citrate, tributyl citrate, acetyl tributyl citrate, triacetin, diethyl phthalate, polyethylene glycols, polysorbates, mono- and diacetylated glycerides, and mixtures thereof.

6. Spheroids according to one of the preceding claims, **characterized in that** they are coated with a water-dispersible outer layer which provides the said spheroids with cohesion during a possible compression step and which ensures breakdown in aqueous medium of the tablet obtained.

7. Spheroids according to Claim 6, **characterized in that** the water-dispersible outer layer consists of an acrylic, vinyl or cellulose polymer.

8. Spheroids according to one of Claims 1 to 7, **characterized in that** the active principle(s) is(are) dispersed in the bulk of the core.

9. Spheroids according to one of Claims 1 to 7, **characterized in that** the active principle(s) is(are) dispersed in the layer containing at least one thermoplastic excipient.

10. Spheroids according to one of Claims 1 to 7, **characterized in that** the active principle(s) is(are) dispersed in the bulk of the core and in the layer containing at least one thermoplastic excipient.

11. Spheroids according to one of Claims 1 to 7, **characterized in that** the active principle(s) is(are) applied to the surface of the core and then coated with a layer containing at least one thermoplastic excipient.

12. Process for the preparation of the spheroids according to one of Claims 1 to 11, **characterized in that** the layer containing at least one thermoplastic excipient, the film containing a polymer material, and optionally the outer layer, are successively deposited on the cores by spraying, in a granulating turbomixer, in a perforated turbomixer, in a fluidized-air bed or by any other appropriate means.

13. Multiparticulate pharmaceutical preparations containing the spheroids according to one of Claims 1 to 11 which can be obtained according to the process of Claim 12.

14. Pharmaceutical preparations according to Claim 13, **characterized in that** they are in the form of gelatin capsules filled with the said spheroids.

15. Pharmaceutical preparations according to Claim 13, **characterized in that** they are in the form of tablets of the said spheroids.

16. Process for manufacturing preparations according to Claim 15, **characterized in that** the tablets are prepared without the addition of a substantial amount of an auxiliary substance in particular less than 5% by weight.

17. Process according to Claim 16, **characterized in that** up to 5% by weight of a lubricant such as talc is added to the spheroids before compression.
